# EUROPEAN PATENT APPLICATION

(11) **EP 4 393 977 A1**
(43) Date of publication of application: **03.07.2024**
(21) Application number: 22860430.2
(22) Date of filing: 22.08.2022
(51) Int. Cl.: C08G 63/78, C08G 63/08, C08K 3/22

(54) **BIODEGRADABLE POLYMER MICROSPHERES AND PREPARATION METHOD THEREFOR**

(30) Priority: 23.08.2021 CN 202110969905
(71) Applicant: Wuhu Weiqiu New Material Technology Co., Ltd., Wuhu, Anhui 241200 (CN)
(72) Inventor: YANG, Guisheng, Shanghai 201108 (CN); ZHAO, Xingke, Hefei, Anhui 238000 (CN); LI, Honglin, Hefei, Anhui 238000 (CN)
(74) Representative: Bardehle Pagenberg Partnerschaft mbB Patentanwälte Rechtsanwälte
(86) International application number: PCT/CN2022/113877
(87) International publication number: WO 2023/025084

(57) **Abstract**

Provided in the present invention are biodegradable polymer microspheres and a preparation method therefor. The preparation method comprises the step of polymerizing a biodegradable polymeric monomer in a mixed solution containing the biodegradable polymeric monomer, a water-soluble polymer and a catalyst. In the present invention, the monomer and the water-soluble polymer are mixed into a solution, and phase separation occurs, after the in situ polymerization of the monomer, to obtain the biodegradable polymer microspheres; the production process is simple and easy to control, without the need for an organic solvent; and the production process is environmentally friendly and the method is suitable for large-scale production.

## Description

### FIELD OF THE INVENTION

The present invention relates to the field of biodegradable polymer material, specifically to biodegradable polymer microspheres and preparation method therefor.

### BACKGROUND

Biodegradable polymer microspheres have wide applications in multiple fields. For example, polylactic acid microspheres have been widely used in immunology, gene therapy, tumor therapy, ophthalmology and many other medical fields as drug controlled release carriers, gene carriers and carriers of polypeptide and protein drugs; polymer microspheres can also be used in 3D printing, powder coatings, ink additives, cosmetic additives and other fields, which is also the potential use of degradable microspheres. The common feature of the prior art of biodegradable polymer microspheres (such as patent applications CN00128164.X, CN201110462504.1 and CN201910103460.X) is that a large amount of organic solvents are used in the preparation process of the polymer microspheres, which causes environmental pollution and harm.

Therefore, there is a need in the art for biodegradable polymer microspheres that do not require an organic solvent in the production process and a preparation method therefor.

### SUMMARY

In order to solve the problems existing in the prior art, the present invention provides biodegradable polymer microspheres and preparation method therefor. The present invention prepares a mixed solution of a biodegradable polymeric monomer and a water-soluble polymer, and triggers in situ polymerization of the monomer to obtain a biodegradable polymer/water-soluble polymer composite material. At this time, the two have undergone phase separation, and the water-soluble polymer is washed away with water to obtain the biodegradable polymer microspheres. The production process is simple and easy to control, without the need for an organic solvent, and the production process is environmentally friendly and the method is suitable for large-scale production.

Specifically, one aspect of the present invention provides a biodegradable polymer microsphere, wherein the biodegradable polymer microsphere comprises a biodegradable polymer, and a particle size of the biodegradable polymer microsphere is ≤ 20µm.

In one or more embodiments, the biodegradable polymer is an aliphatic polyester, such as polylactic acid, polycaprolactone, polyglycolide acid, lactide-caprolactone copolymer, lactide-glycolide copolymer, caprolactone-glycolide copolymer, or lactide-caprolactone-glycolide copolymer.

In one or more embodiments, the particle size of the biodegradable polymer microsphere ranges from 0.5µm to 15µm.

In one or more embodiments, a number of the biodegradable polymer microsphere with a particle size between 1µm to 6µm accounts for more than or equal to 80% of a total number of the biodegradable polymer microsphere.

In one or more embodiments, the biodegradable polymer microsphere further comprises an antioxidant.

In one or more embodiments, the antioxidant is selected from one or more of antioxidant 168 (tri(2,4-di-tert-butylphenyl)phosphite), antioxidant 1010 (pentaerythritol tetrakis[P-(3,5-di-tert-butyl-4-hydroxyphenyl)propionate]), antioxidant 1098 (N,N'-bis-(3-(3,5-di-tert-butyl-4-hydroxyphenyl)propionyl)hexamethylenediamine), antioxidant 626 (bis(2,4-di-tert-butylphenyl)pentaerythritol diphosphite), and antioxidant THP-24 (bis(2,4-di-tert-butylphenyl)pentaerythritol diphosphite).

In one or more embodiments, in the biodegradable polymer microsphere, a mass ratio of the biodegradable polymer to the antioxidant is 100: (0.1-1).

In one or more embodiments, the biodegradable polymer microsphere further comprises a functional material.

In one or more embodiments, the functional material is selected from an inorganic functional powder and a drug.

In one or more embodiments, the inorganic functional powder is selected from one or more of graphene, graphite, carbon nanotubes, and magnetic nanoparticles.

In one or more embodiments, the functional material is the drug, such as erythromycin, ibuprofen, clindamycin, amoxicillin, cefradine, acetylspiramycin, azithromycin, oryzanol, etc.

In one or more embodiments, in the biodegradable polymer microsphere, a mass ratio of the biodegradable polymer to the functional material is 100: (1-20).

Another aspect of the present invention provides a method for preparing a biodegradable polymer microsphere, wherein the method comprises a step of polymerizing a biodegradable polymeric monomer in a mixed solution comprising the biodegradable polymeric monomer, a water-soluble polymer, and a catalyst, and the biodegradable polymer microsphere is preferably as described in any one of the embodiments herein.

In one or more embodiments, the biodegradable polymeric monomer is a monomer of an aliphatic polyester, preferably selected from one or more of aliphatic lactides and aliphatic lactones, such as one or more of lactide, caprolactone, and glycolide.

In one or more embodiments, the water-soluble polymer is selected from one or more of polyoxyethylene-polyoxypropylene ether block copolymer, polyacrylamide, polyvinylpyrrolidone, polyethylene glycol, polypropylene glycol, poly(vinyl alcohol), poly(acrylic acid), poly(methacrylic acid) and methyl cellulose.

In one or more embodiments, in the mixed solution, the mass ratio of the biodegradable polymeric monomer to the water-soluble polymer is 100: (20-300).

In one or more embodiments, the catalyst is selected from a metal, a Lewis acid, a Lewis base, an organometallic compound and a metal salt, such as one or more of Fe, Cu, Zn, Co, an alkali metal salt, a Schiff base, dibutyl magnesium, an aluminum alkoxide, Ti[OCH(CH₃)₂]₄, Sn(Oct)₂, SnCl₄, Sn(C₆H₅)₄, Sn(Oct)₂/CH₃(CH₂)₁₁OH and Sn(Oct)₂/P(C₆H₅)₃.

In one or more embodiments, in the mixed solution, the mass ratio of the biodegradable polymeric monomer to the catalyst is 100: (0.1-2).

In one or more embodiments, the mixed solution further comprises an antioxidant and/or a functional material.

In one or more embodiments, the reaction temperature of polymerization is 90-180°C.

In one or more embodiments, the method comprises the following steps:
S1. preparing a liquid monomer: taking a biodegradable polymeric monomer that is in liquid form at ordinary temperature for later use; or heating and melting a biodegradable polymeric monomer that is in solid form at ordinary temperature to obtain a liquid monomer;
S2. preparing the mixed solution: adding the water-soluble polymer into the liquid monomer, and completely dissolving the water-soluble polymer into the liquid monomer to obtain the mixed solution;
S3. polymerization: adding the catalyst into the mixed solution, letting the solution stand at reaction temperature to promote polymerization of the monomer, and obtaining a mixture of the biodegradable polymer microsphere and the water-soluble polymer;
S4. post-treatment: post-treating the mixture to obtain the biodegradable polymer microsphere.

### BRIEF DESCRIPTION OF THE FIGURES

Figure 1 shows the SEM images of the polylactic acid microspheres prepared in Examples 2, 3, 4, and 5 of the present invention under a scanning electron microscope. In Figure 1, a, b, c, and d respectively shows the morphology of the polylactic acid microspheres prepared in Examples 2, 3, 4, and 5. The left column shows the SEM images with a magnification of 1000 times, and the right column shows the SEM images with a magnification of 5000 times.
Figure 2 shows the particle size distribution diagrams of the polylactic acid microspheres prepared in Examples 2, 3, 4, and 5 of the present invention. In Figure 2, a, b, c, and d respectively shows the particle size distribution diagram of the polylactic acid microspheres prepared in Examples 2, 3, 4, and 5.
Figure 3 shows the SEM image of the polylactic acid microspheres prepared in Example 6 of the present invention.
Figure 4 shows the SEM image of the polylactic acid microspheres prepared in Example 7 of the present invention.
Figure 5 shows the SEM image of the polylactic acid microspheres prepared in Example 8 of the present invention.
Figure 6 shows the SEM image of the polylactic acid microspheres prepared in Example 9 of the present invention.
Figure 7 shows the SEM image of the polycaprolactone microspheres prepared in Example 10 of the present invention.
Figure 8 shows the SEM image of the polyglycolide acid microspheres prepared in Example 11 of the present invention.
Figure 9 shows the SEM image of the magnetic L-lactide-caprolactone copolymer microspheres prepared in Example 12 of the present invention.
Figure 10 shows the particle size distribution diagrams of the microspheres prepared in Examples 6-12 of the present invention. In Figure 10, a, b, c, d, e, f, and g shows the particle size distribution diagram of the microspheres prepared in Examples 6, 7, 8, 9, 10, 11, and 12, respectively.
Figure 11 shows DSC heating curves of the polylactic acid microspheres prepared in Examples 1 and 5 and poloxamer 407.
Figure 12 shows UV absorption spectra of the erythromycin-loaded polylactic acid microspheres prepared in Examples 13, 14 and 15 and erythromycin.

### DETAILED DESCRIPTION

The embodiments of the present invention are described below, and those skilled in the art can easily understand other advantages and functions of the present invention from the disclosure of this specification. Any person skilled in the art can modify the microspheres of the present invention to obtain biodegradable polymer microspheres with certain functions without departing from the spirit and scope of the present invention, which should still be covered by the claims of the present invention. The present invention may be practiced or applied in various other embodiments, and the details in this specification can be modified based on different perspectives and applications without deviating from the spirit of the present invention. It should be noted that the features of the embodiments and Examples of the present invention can be combined with each other as long as there is no contradiction.

In order to enable those skilled in the art to understand the features and effects of the present disclosure, the following is a general description and definition of terms and words mentioned in the specification and claims. Unless otherwise specified, all technical and scientific terms used herein are intended to be the ordinary meaning of the knowledge of the present disclosure by those skilled in the art, and in case of a conflict, the definition of this specification shall prevail.

The theories or mechanisms described and disclosed herein, whether right or wrong, should not limit the scope of the present disclosure in any way, that is to say the present disclosure can be practiced without limitation to any particular theory or mechanism.

As used herein, the terms "comprising", "including", "containing" and the like encompass terms "consisting essentially of" and "consisting of". For example, where it is disclosed herein that "A comprises B and C", "A consists essentially of B and C" and "A consists of B and C" should be considered to be disclosed herein.

In the present invention, all features, such as numerical values, quantities, amounts and concentrations, which are defined by numerical ranges or percentage ranges, are only for the sake of simplicity and convenience. Accordingly, the recitation of numerical ranges or percentage ranges shall be construed as covering and specifically disclosing all possible sub-ranges and individual values (including integers and fractions) in the range.

In the present invention, unless otherwise specified, a percentage refers to a mass percentage, and a proportion refers to a mass ratio.

In the present invention, when embodiments or Examples are described, it should be understood that they are not intended to limit the invention to these embodiments or Examples. On the contrary, all alternatives, improvements and equivalents of the methods and materials described in the present invention can be covered within the scope defined by the claims.

Herein, for the sake of brevity of description, all possible combinations of various technical features in the various embodiments or Examples are not described. Therefore, as long as there is no contradiction in the combination of these technical features, the various technical features in the various embodiments or Examples can be combined in any combination, and all possible combinations should be considered to be within the scope of this specification.

The present invention finds that by preparing a biodegradable polymeric monomer with a water-soluble polymer into a liquid mixture (also called a mixed solution), and enabling the monomer to polymerize in the presence of the water-soluble polymer, a biodegradable polymer formed after polymerization occurs phase separation from the water-soluble polymer. The precipitated biodegradable polymer cannot form continuous phase and forms into microspheres. The water-soluble polymer is washed away by water to obtain the biodegradable polymer microspheres, so that the biodegradable polymer microspheres can be prepared in an environmentally friendly and controllable method without an organic solvent.

The biodegradable polymer microsphere of the present invention comprises a biodegradable polymer. Usually, the mass of the biodegradable polymer accounts for more than or equal to 60%, such as more than or equal to 70%, more than or equal to 80%, or more than or equal to 90%, of the total mass of the biodegradable polymer microsphere of the present invention.

In the present invention, the biodegradable polymer refers to a polymer whose molecular chain can be cleaved (e.g., hydrolyzed) by enzymes or microorganisms (e.g., bacteria, mold, algae, etc.) under certain conditions (e.g., certain humidity, temperature, pH, and/or oxygen concentration). The biodegradable polymer typically comprises one or more biodegradable chemical bonds selected from ester bond (such as aliphatic ester bond), ether bond (such as aliphatic ether bond), amide group (such as peptide bond), carbamate bond, nitrogen-phosphorus double bond (N=P), etc. The biodegradable polymer includes, but is not limited to, polyester (such as aliphatic polyester), polyether (such as aliphatic polyether), polyester ether (such as aliphatic polyester ether), polyamide (such as polyamino acid), poly(ortho ester), polycarbonate, polyanhydride, polyphosphazene, polyurethane, etc. In some embodiments, the biodegradable polymer comprised in the biodegradable polymer microsphere of the present invention is a biodegradable aliphatic polyester, such as polylactic acid, polycaprolactone, polyglycolide acid, lactide-caprolactone copolymer, lactide-glycolide copolymer, caprolactone-glycolide copolymer, lactide-caprolactone-glycolide copolymer, etc.

The particle size of the biodegradable polymer microsphere of the present invention is usually ≤ 20µm. In some embodiments, the particle size of the biodegradable polymer microsphere of the present invention is not greater than 18µm, 15µm, 13µm, 10µm, 9µm, or 8µm. The particle size of the biodegradable polymer microsphere of the present invention is usually ≥0.1µm. In some embodiments, the particle size of the biodegradable polymer microsphere of the present invention is not less than 0.2µm, 0.5µm, 1µm, or 2µm. In some embodiments, the particle size of the biodegradable polymer microsphere of the present invention is 0.5-15µm.

In some preferred embodiments, in the biodegradable polymer microsphere of the present invention, the number of the biodegradable polymer microsphere with a particle size of 1-6µm accounts for more than or equal to 80% of the total number of the biodegradable polymer microsphere. In some embodiments, in the biodegradable polymer microsphere of the present invention, the number of the biodegradable polymer microsphere with the particle size of 1-6µm accounts for more than or equal to 80% of the total number of the biodegradable polymer microsphere. In some embodiments, the particle size of the biodegradable polymer microsphere of the present invention is 1-13µm, and the number of the biodegradable polymer microsphere with a particle size of 1-4µm accounts for more than or equal to 88% of the total number of the microsphere. In some embodiments, the particle size of the biodegradable polymer microsphere of the present invention is 2-9µm, and the number of the biodegradable polymer microsphere with a particle size of 2-6µm accounts for more than or equal to 85% of the total number of the microsphere. In some embodiments, the particle size of the biodegradable polymer microsphere of the present invention is 1-8µm, and the number of the biodegradable polymer microsphere with a particle size of 3-6µm accounts for more than or equal to 80% of the total number of the microsphere. In some embodiments, the particle size of the biodegradable polymer microsphere of the present invention is 1-13 µm, the number of the biodegradable polymer microsphere with a particle size of 1-3µm accounts for more than or equal to 75% of the total number of the microsphere, and the number of the biodegradable polymer microsphere with a particle size of 1-6µm accounts for more than or equal to 80% of the total number of the microsphere. In some embodiments, the particle size of the biodegradable polymer microsphere of the present invention is 1-3µm, and the number of the biodegradable polymer microsphere with a particle size of 1-2µm accounts for more than or equal to 80% of the total number of the microsphere.

Some biodegradable polymers (such as polylactic acid) polymerize at high temperature. In order to prevent oxidation and obtain higher molecular weight products, an appropriate amount of antioxidant can be added to the reaction system. Therefore, in some embodiments, the biodegradable polymer microsphere of the present invention further comprises an antioxidant. The antioxidant suitable for the present invention is preferably selected from one or more of antioxidant 168, antioxidant 1010, antioxidant 1098, antioxidant 626, and antioxidant THP-24. When the biodegradable polymer microsphere of the present invention comprises the antioxidant, the mass ratio of the biodegradable polymer to the antioxidant comprised in the biodegradable polymer microsphere can be 100: (0.1-1), such as 100:0.2, 100:0.5, 100:0.6, 100:0.7, 100:0.8, 100:0.9. When the biodegradable polymer microsphere is applied in the field of biomedicine, the biodegradable polymer microsphere of the present invention preferably does not comprise the antioxidant, or only comprises a small amount of the antioxidant, preferably non-toxic antioxidant.

In some embodiments, the biodegradable polymer microsphere of the present invention further comprises one or more functional materials with specific functions. The functional material does not comprise the aforementioned antioxidant. The functional material suitable for the present invention includes, but is not limited to, an immunological material, a drug controlled release material, a magnetic material, a conductive material, a drug, a dye, a fluorescent molecule, a pH responsive material, etc. The functional material suitable for the present invention may be an organic material, an inorganic material, or an organic-inorganic composite material. The size of the functional material suitable for the present invention may be nanoscale (1-100nm) or submicroscale (100-1000nm). In some embodiments, the functional material is an inorganic functional powder, such as graphene, graphite, carbon nanotubes, magnetic nanoparticles, and the like. Examples of the magnetic nanoparticles include, but are not limited to, Co ferrite nanoparticles. In some embodiments, the functional material is the drug, such as erythromycin, ibuprofen, clindamycin, amoxicillin, cefradine, acetylspiramycin, azithromycin, oryzanol, etc. When the biodegradable polymer microsphere of the present invention comprises the functional material, the mass ratio of the biodegradable polymer to the functional material comprised in the biodegradable polymer microsphere can be 100: (1-20), such as 100:2, 100:5, 100:10, 100:15. Controlling the mass ratio of the functional material to the biodegradable polymer below or equal to 20:100 is beneficial for microspheres formation.

In some embodiments, the biodegradable polymer microsphere of the present invention comprises a biodegradable polymer, an optional antioxidant, an optional functional material. In some embodiments, the biodegradable polymer microsphere of the present invention mainly consists of a biodegradable polymer, an optional antioxidant, and an optional functional material. The total mass of the biodegradable polymer, the optional antioxidant, and the optional functional material may be more than or equal to 80%, more than or equal to 90%, more than or equal to 95%, more than or equal to 98%, more than or equal to 99%, more than or equal to 99.5%, or 100% of the total mass of the biodegradable polymer microsphere.

The biodegradable polymer microsphere of the present invention may comprise trace amounts of catalyst residues.

The biodegradable polymer in the biodegradable polymer microsphere of the present invention may be grafted with the water-soluble polymer described herein (e.g., polyoxyethylene-polyoxypropylene ether block copolymer).

The method for preparing the biodegradable polymer microsphere of the present invention comprises a step of polymerizing a biodegradable polymeric monomer in a mixed solution comprising the biodegradable polymeric monomer, a water-soluble polymer and a catalyst.

The biodegradable polymeric monomer suitable for the present invention may be the monomer known in the art for preparing the biodegradable polymer. In some embodiments, the biodegradable polymeric monomer is a monomer of a biodegradable aliphatic polyester, preferably selected from one or more of aliphatic lactides and aliphatic lactones, such as one or more of lactide (such as L-lactide), caprolactone, glycolide, etc. When multiple biodegradable polymeric monomers are used, the ratio between each monomer is not particularly limited.

In the present invention, the water-soluble polymer (water soluble macromolecular, also referred to herein as a water-soluble polymer additive) refers to a macromolecule that can dissolve or swell in water to form an aqueous solution or dispersion, and has a molecular weight of 300g/mol or more. The water-soluble polymer suitable for the present invention comprises one or more selected from polyoxyethylene-polyoxypropylene ether block copolymer, polyacrylamide, polyvinylpyrrolidone, polyethylene glycol, polypropylene glycol, poly(vinyl alcohol), poly(acrylic acid), poly(methacrylic acid), methyl cellulose, etc. In some embodiments, the water-soluble polymer is selected from one or more of polyoxyethylene-polyoxypropylene ether block copolymer, polyvinylpyrrolidone, polyethylene glycol and methyl cellulose. The preferred molecular weight of the water-soluble polymer is 300-50000 g/mol, such as 1000 g/mol, 2000 g/mol, 5000 g/mol, 10000 g/mol, 15000 g/mol, 20000 g/mol, 25000 g/mol, 30000 g/mol, 40000 g/mol. The viscosity of methyl cellulose suitable for the present invention is preferably 10000-100000 mPa s, such as 20000 mPa s, 40000 mPa s, 80000 mPa s. The useful polyoxyethylene-polyoxypropylene ether block copolymer comprises diblock copolymer, triblock copolymer, and copolymer with more blocks, such as polyethylene glycol)-block-poly(propylene glycol)-block-poly(ethylene glycol) (PEG-b-PPG-b-PEG). In PEG-b-PPG-b-PEG, the content of PEG segment can be 50-90wt%, preferably 60-80wt%, such as 70-75wt%. Examples of the PEG-b-PPG-b-PEG comprise poloxamer 407. In some embodiments, the water-soluble polymer used in the present invention is selected from one or more of the following water-soluble polymer: polyoxyethylene-polyoxypropylene ether block copolymer (such as PEG-b-PPG-b-PEG) with a molecular weight of 5000-20000 g/mol, such as 9000-15000 g/mol, or methyl cellulose with a viscosity of 40000-100000 mPa·s, such as 80000-100000 mPa s, or polyvinylpyrrolidone with a molecular weight of 5000-10000 g/mol, such as 7000-9000 g/mol, or polyethylene glycol with a molecular weight of 5000-15000 g/mol, such as 8000-12000 g/mol. Controlling the molecular weight or viscosity of the water-soluble polymer within the above range is beneficial to phase separation after polymerization of the biodegradable polymeric monomer to obtain microspheres with the particle size meeting the requirements of the present invention.

In the mixed solution used for polymerization of the present invention, the mass ratio of the biodegradable polymeric monomer to the water-soluble polymer is 100: (20-300), such as 100:20, 100:30, 100:40, 100:50, 100:60, 100:70, 100:80, 100:90, 100:100, 100:150, 100:20, 100:250, 100:300. The larger the mass proportion of the water-soluble polymer, the smaller the particle size of the biodegradable polymer microsphere prepared. In some embodiments, the mass ratio of the biodegradable polymeric monomer to the water-soluble polymer is 100: (20-100). In these embodiments, the particle size of the biodegradable polymer microsphere can be controlled to be 0.5-15µm, and the number of the biodegradable polymer microsphere with the particle size of 1-6µm can account for more than or equal to 80% of the total number of the biodegradable polymer microsphere.

In some embodiments, the method of the present invention for preparing the biodegradable polymer microsphere further comprises preparing the mixed solution comprising the biodegradable polymeric monomer and the water-soluble polymer, and then adding a catalyst to the mixed solution to polymerize the biodegradable polymeric monomer.

In the present invention, in an embodiment where the biodegradable polymeric monomer is liquid (for example, the monomer is caprolactone) at ambient temperature, such as ordinary temperature (25°C), the biodegradable polymeric monomer and the water-soluble polymer can be mixed uniformly at ambient temperature, such as ordinary temperature or heating conditions, to obtain the mixed solution. For example, the monomer in a liquid state at ordinary temperature and the water-soluble polymer may be mixed and stirred for 20-40 min to obtain the mixed solution.

In the present invention, in an embodiment where the biodegradable polymeric monomer comprises a monomer that is solid at ambient temperature, such as ordinary temperature (25°C) (such as L-lactide, glycolide), the temperature of the mixed solution used for polymerization is not lower than the melting point of the monomer that is solid at ambient temperature. If there are multiple monomers that are solid at ambient temperature, the temperature of the mixed solution shall not be lower than the melting point of the solid monomer with the highest melting point. The biodegradable polymeric monomer can be heated to melt, preferably kept for a period of time (e.g. 10-20 min) to obtain a liquid monomer, and then mixed evenly with the water-soluble polymer to obtain the mixed solution. For example, the heated and melted liquid monomer and the water-soluble polymer may be mixed and stirred for 20-40 min to obtain the mixed solution.

The mixed solution comprising the biodegradable polymeric monomer and the water-soluble polymer can also comprises a functional material. In general, the functional material is introduced into the mixed solution before the catalyst is added. For example, after the liquid monomer and the water-soluble polymer are uniformly mixed, the functional material is added and the solution is continued to be uniformly mixed. Suitable functional material and amount thereof are as described above.

In the present invention, after adding the catalyst to the mixed solution comprising the biodegradable polymeric monomer and the water-soluble polymer, the biodegradable polymeric monomer is subjected to ring-opening polymerization. Ring-opening polymerization can be anionic, cationic, or coordinated ring-opening polymerization. The catalyst used in the present invention can be a metal, a Lewis acid, a Lewis base, an organometallic compound and a metal salt, such as one or more of Fe, Cu, Zn, Co, an alkali metal salt, a Schiff base, dibutyl magnesium, an aluminum alkoxide, Ti[OCH(CH₃)₂]₄, Sn(Oct)₂, SnCl₄, Sn(C₆H₅)₄, Sn(Oct)₂/CH₃(CH₂)₁₁OH and Sn(Oct)₂/P(C₆H₅)₃. Herein, Sn(Oct)₂/CH₃(CH₂)₁₁OH refers to a composition of Sn(Oct)₂ and CH₃(CH₂)₁₁OH, where a molar ratio of Sn(Oct)₂ to CH₃(CH₂)₁₁OH can be from 2:1 to 1:2, for example 1:1. Herein, Sn(Oct)₂/P(C₆H₅)₃ refers to a composition of Sn(Oct)₂ and P(C₆H₅)₃, where a molar ratio of Sn(Oct)₂ to P(C₆H₅)₃ can be from 2:1 to 1:2, such as 1:1. In some embodiments, the catalyst is selected from one or more of Ti[OCH(CH₃)₂]₄, Sn(Oct)₂, Sn(Oct)₂/CH₃(CH₂)₁₁OH and Sn(Oct)₂/P(C₆H₅)₃. In the reaction system, a feeding mass ratio of the biodegradable polymeric monomer to the catalyst is preferably 100: (0.1-2), for example, 100:0.2, 100:0.3, 100:0.4, 100:0.5, or 100:1. The active end group of the water-soluble polymer will consume part of the catalyst. When the content of the water-soluble polymer is relatively high or the molecular weight of the water-soluble polymer is relatively low, the amount of the catalyst to be added need to be relatively high. When the content of the water-soluble polymer is relatively low or the molecular weight of the water-soluble polymer is relatively high, the amount of the catalyst to be added can be relatively low.

In the present invention, while adding the catalyst to the mixed solution comprising the biodegradable polymeric monomer and the water-soluble polymer, an antioxidant can be optionally added. The suitable antioxidant and amount thereof are as described above.

In the present invention, the temperature of the reaction system in the polymerization reaction is 90-180°C, such as 90°C, 95°C, 100°C, 105°C, 110°C, 115°C, 120°C, 125°C, 130°C, 140°C, 150°C, 160°C. The appropriate reaction temperature is selected based on the type of the biodegradable polymeric monomer. For example, when the monomer is L-lactide, the reaction temperature is preferably 130-180°C, such as 140-150°C; when the monomer is glycolide, the reaction temperature is preferably 90-130°C, such as 110-120°C; when the monomer is caprolactone, the reaction temperature is preferably 110-140°C, such as 120-130°C; when the monomers are L-lactide and caprolactone, the reaction temperature is preferably 110-140°C, such as 139-140°C. The reaction is usually carried out in a standing state, which facilitates phase separation. The reaction time can be 0.5h-24h, such as 1h, 1.5h, 2h, 2.5h, 3h, 5h, 10h, 24h. In the reaction process, the reaction system is gradually changed into a milky white solid from a transparent and homogeneous solution. When the reaction system is completely cured, it can be considered as the end of the reaction.

After the polymerization reaction is completed, a product system comprising the biodegradable polymer microsphere and the water-soluble polymer is obtained. The method of the present invention for preparing the biodegradable polymer microsphere may further comprise carrying out post-treatment on the product system, and separating to obtain the biodegradable polymer microsphere. For example, post-treatment can comprise cooling the product system to ambient temperature, such as ordinary temperature, followed by water washing, filtration, and drying. The number of times of water washing may be, for example, 5-8 times.

In some embodiments, the biodegradable polymer microsphere of the present invention comprises the following materials in parts by weight:
a biodegradable polymeric monomer: 100 parts;
a water-soluble polymer additive: 20-300 parts, such as 20-100 parts;
an antioxidant: 0.1-1 parts, such as 0.5-1 parts; and
a catalyst: 0.1-2 parts, such as 0.1-0.5 parts.

Furthermore, the biodegradable polymer of the present invention may further comprise 1-20 parts by weight of a functional material with respect to 100 parts by weight of the biodegradable polymeric monomer.

In some embodiments, the raw material required for preparing the biodegradable polymer microsphere of the present invention comprises the following materials in parts by weight, or consists of the following materials in parts by weight:
the biodegradable polymeric monomer: 100 parts;
the water-soluble polymer additive: 20-300 parts, such as 20-100 parts;
the antioxidant: 0.1-1 parts, such as 0.5-1 parts;
the catalyst: 0.1-2 parts, such as 0.1-0.5 parts; and
the optional functional material: 1-20 parts.

Furthermore, the biodegradable polymeric monomer comprises one or more of lactide, caprolactone, and glycolide; and further, the lactide is L-lactide.

Furthermore, the water-soluble polymer additive is selected from one or more of polyoxyethylene-polyoxypropylene ether block copolymer, polyacrylamide, polyvinylpyrrolidone, or polyethylene glycol.

Furthermore, the water-soluble polymer additive is selected from one or more of polyoxyethylene-polyoxypropylene ether block copolymer, methyl cellulose, polyvinylpyrrolidone, or polyethylene glycol.

Furthermore, the antioxidant is selected from one or more of antioxidant 168, antioxidant 1010, antioxidant 1098, antioxidant 626, or antioxidant THP-24.

Furthermore, the catalyst is selected from a metal, a Lewis acid, a Lewis base, an organometallic compound and a metal salt, such as one or more of Fe, Cu, Zn, Co, an alkali metal salt, a Schiff base, dibutyl magnesium, an aluminum alkoxide, Ti[OCH(CH₃)₂]₄, Sn(Oct)₂, SnCl₄, Sn(C₆H₅)₄, Sn(Oct)₂/CH₃(CH₂)₁₁OH and Sn(Oct)₂/P(C₆H₅)₃; and further, the catalyst is selected from one or more of Ti[OCH(CH₃)₂]₄, Sn(Oct)₂, Sn(Oct)₂/CH₃(CH₂)₁₁OH and Sn(Oct)₂/P(C₆H₅)₃.

Furthermore, the functional material is an inorganic functional powder, and the inorganic functional powder, for example, is at least one of graphene, graphite, carbon nanotubes, and magnetic nanoparticles, etc.

Furthermore, the functional material is a drug, and the drug, for example, is at least one of erythromycin, ibuprofen, clindamycin, amoxicillin, cefradine, acetylspiramycin, azithromycin, oryzanol, etc.

In some embodiments, the preparation method for the biodegradable polymer microsphere of the present invention is carried out by dissolving water-soluble polymer additive as continuous phase in the biodegradable polymeric monomer, and adding the antioxidant and the catalyst to promote the monomer to polymerize into the biodegradable polymer microsphere. Due to the insolubility of the water-soluble polymer additive in the biodegradable polymer, the water-soluble polymer additive precipitates and occurs phase separation from the biodegradable polymer during the polymerization of the monomer, causing the biodegradable polymer to disperse into microspheres.

In some embodiments, the preparation method for the biodegradable polymer microsphere of the present invention comprises the following steps:
S 1. preparing a liquid monomer: taking a biodegradable polymeric monomer that is in liquid form at ordinary temperature for later use; or heating and melting a biodegradable polymeric monomer that is solid at ordinary temperature to obtain the liquid monomer;
S2. preparing a mixed solution: adding a water-soluble polymer into the liquid monomer, and completely dissolving the water-soluble polymer into the liquid monomer to obtain the mixed solution;
S3. polymerization: adding a catalyst into the mixed solution, letting the solution stand at reaction temperature to promote the polymerization of the monomer, and obtaining a mixture of the biodegradable polymer microsphere and the water-soluble polymer;
S4. post-treatment: post-treating the mixture to obtain the biodegradable polymer microsphere.

Furthermore, the preparation method specifically comprises the following steps:
S 1. preparing the liquid monomer: taking the biodegradable polymeric monomer that is in liquid form at ordinary temperature for later use; or heating and melting the biodegradable polymeric monomer that is solid at room temperature, and maintaining the temperature for 10-20 min to obtain the liquid monomer;
S2. preparing the mixed solution: adding the water-soluble polymer additive into the liquid monomer, continuously stirring for 20-40 min, and completely dissolving the water-soluble polymer additive into the liquid monomer to obtain the mixed solution;
S3. polymerization: adding the antioxidant and the catalyst into the mixed solution, letting the solution stand at reaction temperature to promote polymerization of the monomer, or stirring to promote polymerization of the monomer, naturally cooling to ordinary temperature, and obtaining a mixture of the biodegradable polymer microsphere and the water-soluble polymer additive;
S4. water washing: washing the mixture with water (e.g. 5-8 times), filtering and drying, and removing the water-soluble polymer additive to obtain the biodegradable polymer microsphere.

Furthermore, after preparing the mixed solution in step S2, the functional material, such as the inorganic functional powder or the drug, can be added for stirring and mixing.

Furthermore, the inorganic functional powder is at least one of graphene, graphite, carbon nanotubes, magnetic nanoparticles, etc.

Furthermore, in the step S3, the monomer polymerization is promoted preferably by standing, for example, by static casting, at the reaction temperature.

The present invention has the following beneficial effects:
the preparation method for the biodegradable polymer microsphere provided by the present invention has the advantages that the process is simple and easy to control, no organic solvent is needed in the preparation process, the pollution is reduced while the cost is reduced, and the large-scale industrial production can be realized;
the preparation method for the biodegradable polymer microspheres of the present invention comprises preparing the biodegradable polymeric monomer and the water-soluble polymer additive into the mixed solution (in some embodiments, the liquid monomer is solvent, the water-soluble polymer additive is solute, and the water-soluble polymer additive is dissolved into the monomer to form solution); the water-soluble polymer additive forms continuous phase on the surface of the monomer, and phase separation occurs to obtain the biodegradable polymer microsphere after in situ polymerization of the monomer (the water-soluble polymer additive is insoluble in the biodegradable polymer, and the biodegradable polymer cannot form continuous phase when precipitated by the water-soluble polymer additive, resulting in phase separation and forming microspheres). The production process is simple and easy to control.

The water-soluble polymer additive can be washed away with water, without the need for organic solvents. The process is simple, the production cost is low, the production process is environmentally friendly, and the industrial prospects are promising.

The invention can prepare biodegradable polymer microspheres with narrow particle size distribution, for example, in some embodiments of the invention, the particle size of the biodegradable polymer microspheres is 0.5-15µm, and the number of the biodegradable polymer microspheres with a particle size of 1-6µm accounts for more than or equal to 80% of the total number of the biodegradable polymer microspheres.

The present invention can prepare biodegradable polymer microspheres loaded with functional materials (such as an inorganic functional powder and a drug), which is beneficial for their applications in immunology, gene therapy, tumor therapy, and other fields.

The present invention will be described below in the form of specific examples. It should be understood that these examples are merely illustrative and are not intended to limit the scope of the present description. Unless otherwise specified, the methods, reagents and materials used in the following examples are conventional in the art. The raw material compounds used in the examples are commercially available.

### Example 1

S1. L-lactide melting: heating 100 parts by mass of solid L-lactide to 100°C until completely melted, and keeping the temperature for 10 min to obtain L-lactide melt;
S2. preparing the mixed solution: adding 20 parts by mass of the water-soluble polymer additive PEG-b-PPG-b-PEG (poloxamer 407, molecular weight: 9840-14600g/mol, the content of PEG segment: 71-75wt%) to the L-lactide melt, stirring and heating to 120°C, continuously stirring at the speed of 100 r/min for 30min, and completely dissolving the poloxamer in the L-lactide melt to obtain the mixed solution;
S3. polymerization: adding 0.5 parts by mass of antioxidant THP-24 and 0.1 parts by mass of catalyst Sn(Oct)₂ to the mixed solution, heating to 150°C to promote the polymerization of L-lactide, letting the solution stand for reaction for 1h, and then naturally cooling to ordinary temperature to obtain a mixture of polylactic acid microspheres and the poloxamer;
S4. water washing: washing the mixture with water for 5 times, filtering and drying, and removing the poloxamer to obtain the polylactic acid microspheres.

### Example 2

S1. L-lactide melting: heating 100 parts by mass of solid L-lactide to 100°C until completely melted, and keeping the temperature for 10 min to obtain L-lactide melt;
S2. preparing the mixed solution: adding 40 parts by mass of the water-soluble polymer additive PEG-b-PPG-b-PEG (poloxamer 407, molecular weight: 9840-14600g/mol, the content of PEG segment: 71-75wt%) to the L-lactide melt, stirring and heating to 120°C, continuously stirring at the speed of 100 r/min for 30min, and completely dissolving the poloxamer in the L-lactide melt to obtain the mixed solution;
S3. polymerization: adding 0.6 parts by mass of antioxidant THP-24 and 0.2 parts by mass of catalyst Sn(Oct)₂ to the mixed solution, heating to 150°C to promote the polymerization of L-lactide, letting the solution stand for reaction for 1h, and then naturally cooling to ordinary temperature to obtain a mixture of polylactic acid microspheres and the poloxamer;
S4. water washing: washing the mixture with water for 5 times, filtering and drying, and removing the poloxamer to obtain the polylactic acid microspheres.

### Example 3

S1. L-lactide melting: heating 100 parts by mass of solid L-lactide to 100°C until completely melted, and keeping the temperature for 10 min to obtain L-lactide melt;
S2. preparing the mixed solution: adding 60 parts by mass of the water-soluble polymer additive PEG-b-PPG-b-PEG (poloxamer 407, molecular weight: 9840-14600g/mol, the content of PEG segment: 71-75wt%) to the L-lactide melt, stirring and heating to 120°C, continuously stirring at the speed of 100 r/min for 30min, and completely dissolving the poloxamer in the L-lactide melt to obtain the mixed solution;
S3. polymerization: adding 0.7 parts by mass of antioxidant THP-24 and 0.3 parts by mass of catalyst Sn(Oct)₂ to the mixed solution, heating to 150°C to promote the polymerization of L-lactide, letting the solution stand for reaction for 1h, and then naturally cooling to ordinary temperature to obtain a mixture of polylactic acid microspheres and the poloxamer;
S4. water washing: washing the mixture with water for 5 times, filtering and drying, and removing the poloxamer to obtain the polylactic acid microspheres.

### Example 4

S1. L-lactide melting: heating 100 parts by mass of solid L-lactide to 100°C until completely melted, and keeping the temperature for 10 min to obtain L-lactide melt;
S2. preparing the mixed solution: adding 80 parts by mass of the water-soluble polymer additive PEG-b-PPG-b-PEG (poloxamer 407, molecular weight: 9840-14600g/mol, the content of PEG segment: 71-75wt%) to the L-lactide melt, stirring and heating to 120°C, continuously stirring at the speed of 100 r/min for 30min, and completely dissolving the poloxamer in the L-lactide melt to obtain the mixed solution;
S3. polymerization: adding 0.8 parts by mass of antioxidant THP-24 and 0.4 parts by mass of catalyst Sn(Oct)₂ to the mixed solution, heating to 150°C to promote the polymerization of L-lactide, letting the solution stand for reaction for 1h, and then naturally cooling to ordinary temperature to obtain a mixture of polylactic acid microspheres and the poloxamer;
S4. water washing: washing the mixture with water for 5 times, filtering and drying, and removing the poloxamer to obtain the polylactic acid microspheres.

### Example 5

S1. L-lactide melting: heating 100 parts by mass of solid L-lactide to 100°C until completely melted, and keeping the temperature for 10 min to obtain L-lactide melt;
S2. preparing the mixed solution: adding 100 parts by mass of the water-soluble polymer additive PEG-b-PPG-b-PEG (poloxamer 407, molecular weight: 9840-14600g/mol, the content of PEG segment: 71-75wt%) to the L-lactide melt, stirring and heating to 120°C, continuously stirring at the speed of 100 r/min for 30min, and completely dissolving the poloxamer in the L-lactide melt to obtain the mixed solution;
S3. polymerization: adding 1 part by mass of antioxidant THP-24 and 0.5 parts by mass of catalyst Sn(Oct)₂ to the mixed solution, heating to 150°C to promote the polymerization of L-lactide, letting the solution stand for reaction for 1h, and then naturally cooling to ordinary temperature to obtain a mixture of polylactic acid microspheres and the poloxamer;
S4. water washing: washing the mixture with water for 5 times, filtering and drying, and removing the poloxamer to obtain the polylactic acid microspheres.

### Example 6

S1. L-lactide melting: heating 100 parts by mass of solid L-lactide to 98°C until completely melted, and keeping the temperature for 15 min to obtain L-lactide melt;
S2. preparing the mixed solution: adding 30 parts by mass of the water-soluble polymer additive methyl cellulose (viscosity: 100000 mPa s) to the L-lactide melt, stirring and heating to 110°C, continuously stirring at the speed of 110 r/min and removing water under vacuum for 20 min, and completely dissolving the methyl cellulose in the L-lactide melt to obtain the mixed solution;
S3. polymerization: adding 0.9 parts by mass of antioxidant 168 and 0.3 parts by mass of catalyst Ti[OCH(CH₃)₂]₄ to the mixed solution, heating to 150°C to promote the polymerization of L-lactide, letting the solution stand for reaction for 1h, and then naturally cooling to ordinary temperature to obtain a mixture of polylactic acid microspheres and the methyl cellulose;
S4. water washing: washing the mixture with water for 6 times, filtering and drying, and removing the methyl cellulose to obtain the polylactic acid microspheres.

### Example 7

S1. L-lactide melting: heating 100 parts by mass of solid L-lactide to 102°C until completely melted, and keeping the temperature for 20 min to obtain L-lactide melt;
S2. preparing the mixed solution: adding 50 parts by mass of the water-soluble polymer additive polyvinylpyrrolidone (molecular weight: 8000g/mol) to the L-lactide melt, stirring and heating to 130°C, continuously stirring at the speed of 120 r/min for 40min, and completely dissolving the polyvinylpyrrolidone in the L-lactide melt to obtain the mixed solution;
S3. polymerization: adding 0.7 parts by mass of antioxidant 1010 and 0.4 parts by mass of catalyst Sn(Oct)₂/CH₃(CH₂)₁₁OH (molar ratio: 1:1) to the mixed solution, heating to 140°C to promote the polymerization of L-lactide, letting the solution stand for reaction for 2h, and then naturally cooling to ordinary temperature to obtain a mixture of polylactic acid microspheres and the polyvinylpyrrolidone;
S4. water washing: washing the mixture with water for 7 times, filtering and drying, and removing the polyvinylpyrrolidone to obtain the polylactic acid microspheres.

### Example 8

S1. L-lactide melting: heating 100 parts by mass of solid L-lactide to 101°C until completely melted, and keeping the temperature for 15 min to obtain L-lactide melt;
S2. preparing the mixed solution: adding 70 parts by mass of the water-soluble polymer additive polyethylene glycol (molecular weight: 10000g/mol) to the L-lactide melt, stirring and heating to 125°C, continuously stirring at the speed of 115 r/min for 25min, and completely dissolving the polyethylene glycol in the L-lactide melt to obtain the mixed solution;
S3. polymerization: adding 0.5 parts by mass of catalyst Sn(Oct)₂/P(C₆H₅)₃ (molar ratio: 1:1) to the mixed solution, heating to 140°C to promote the polymerization of L-lactide, letting the solution stand for reaction for 2h, and then naturally cooling to ordinary temperature to obtain a mixture of polylactic acid microspheres and the polyethylene glycol;
S4. water washing: washing the mixture with water for 8 times, filtering and drying, and removing the polyethylene glycol to obtain the polylactic acid microspheres.

### Example 9

S1. L-lactide melting: heating 100 parts by mass of solid L-lactide to 99°C until completely melted, and keeping the temperature for 14 min to obtain L-lactide melt;
S2. preparing the mixed solution: adding 90 parts by mass of the water-soluble polymer additive polyethylene glycol (molecular weight: 10000g/mol) to the L-lactide melt, stirring and heating to 115°C, continuously stirring at the speed of 110 r/min for 35min, and completely dissolving the polyethylene glycol in the L-lactide melt to obtain the mixed solution;
S3. polymerization: adding 0.3 parts by mass of catalyst Sn(Oct)₂/CH₃(CH₂)₁₁OH (molar ratio: 1:1) to the mixed solution, heating to 140 °C to promote the polymerization of L-lactide, letting the solution stand for reaction for 2h, and then naturally cooling to ordinary temperature to obtain a mixture of polylactic acid microspheres and the polyethylene glycol;
S4. water washing: washing the mixture with water for 8 times, filtering and drying, and removing the polyethylene glycol to obtain the polylactic acid microspheres.

### Example 10

S1. preparing caprolactone: taking 100 parts by mass of liquid caprolactone at room temperature for later use;
S2. preparing the mixed solution: adding 70 parts by mass of the water-soluble polymer additive polyethylene glycol (molecular weight: 10000g/mol) to the caprolactone, continuously stirring at the speed of 110 r/min for 35min, and completely dissolving the polyethylene glycol in the caprolactone to obtain the mixed solution;
S3. polymerization: adding 0.3 parts by mass of catalyst Sn(Oct)₂ to the mixed solution, heating to 130°C to promote the polymerization of caprolactone, letting the solution stand for reaction for 2h, and then naturally cooling to ordinary temperature to obtain a mixture of polycaprolactone and the polyethylene glycol;
S4. water washing: washing the mixture with water for 8 times, filtering and drying, and removing the polyethylene glycol to obtain the polycaprolactone (PCL) microspheres.

### Example 11

S 1. glycolide melting: heating 100 parts by mass of solid glycolide to 85 °C until completely melted, and keeping the temperature for 14 min to obtain glycolide melt;
S2. preparing the mixed solution: adding 60 parts by mass of the water-soluble polymer additive polyethylene glycol (molecular weight: 10000g/mol) to the glycolide melt, stirring and heating to 95 °C, continuously stirring at the speed of 110 r/min for 35min, and completely dissolving the polyethylene glycol in the glycolide melt to obtain the mixed solution;
S3. polymerization: adding 0.4 parts by mass of catalyst Sn(Oct)₂ to the mixed solution, heating to 110 °C to promote the polymerization of glycolide, letting the solution stand for reaction for 2h, and then naturally cooling to ordinary temperature to obtain a mixture of polyglycolide acid microspheres and the polyethylene glycol;
S4. water washing: washing the mixture with water for 8 times, filtering and drying, and removing the polyethylene glycol to obtain the polyglycolide acid (PGA) microspheres.

### Example 12

S 1. preparing the liquid monomer: dissolving 50 parts by mass of solid L-lactide in 50 parts by mass of caprolactone, heating to 101°C until completely melted, and keeping the temperature for 15 min to obtain a mixed melt of L-lactide and caprolactone;
S2. preparing the mixed solution: adding 50 parts by mass of the water-soluble polymer additive polyethylene glycol (molecular weight: 10000g/mol) and 20 parts by mass of polyvinylpyrrolidone (molecular weight: 8000g/mol) to the mixed melt of L-lactide and caprolactone, stirring and heating to 125°C, continuously stirring at the speed of 115 r/min for 25min, and completely dissolving the polyethylene glycol and the polyvinylpyrrolidone in the mixed melt of L-lactide and caprolactone to obtain the mixed solution;
S3. adding 10 parts by mass of Co ferrite nanoparticles, continuously stirring at the speed of 115 r/min for 25min, and fully mixing;
S4. polymerization: adding 0.4 parts by mass of antioxidant 1098, 0.3 parts by mass of antioxidant 1010, 0.2 parts by mass of catalyst Sn(Oct)₂/P(C₆H₅)₃ (molar ratio: 1:1) and 0.4 parts by mass of Sn(Oct)₂ to the material obtained in S3, heating to 140°C to promote the copolymerization of L-lactide and caprolactone, letting the system stand for reaction for 1.5h, and then naturally cooling to ordinary temperature to obtain a mixture of magnetic L-lactide-caprolactone copolymer microspheres, the polyethylene glycol and the polyvinylpyrrolidone;
S5. water washing: washing the mixture with water for 8 times, filtering and drying, and removing the polyethylene glycol and the polyvinylpyrrolidone to obtain the magnetic L-lactide-caprolactone copolymer microspheres.

### Example 13

S 1. L-lactide melting: heating 100 parts by mass of solid L-lactide to 100°C until completely melted, and keeping the temperature for 10 min to obtain L-lactide melt;
S2. preparing the mixed solution: adding 40 parts by mass of the water-soluble polymer additive PEG-b-PPG-b-PEG (poloxamer 407, molecular weight: 9840-14600g/mol, the content of PEG segment: 71-75wt%) to the L-lactide melt, stirring and heating to 120°C, continuously stirring at the speed of 100 r/min for 30min, and completely dissolving the poloxamer in the L-lactide melt to obtain the mixed solution;
S3. adding 5 parts by mass of erythromycin, continuously stirring at the speed of 115 r/min for 25min, and fully mixing;
S4. polymerization: adding 0.2 parts by mass of catalyst Sn(Oct)₂ to the material obtained from S3, heating to 150°C to promote the polymerization of L-lactide, letting the system stand for reaction for 1h, and then naturally cooling to ordinary temperature to obtain a mixture of erythromycin, polylactic acid microspheres, and the poloxamer;
S4. water washing: washing the mixture with water for 5 times, filtering and drying, and removing the poloxamer to obtain erythromycin-loaded polylactic acid microspheres.

### Example 14

S1. L-lactide melting: heating 100 parts by mass of solid L-lactide to 100°C until completely melted, and keeping the temperature for 10 min to obtain L-lactide melt;
S2. preparing the mixed solution: adding 40 parts by mass of the water-soluble polymer additive PEG-b-PPG-b-PEG (poloxamer 407, molecular weight: 9840-14600g/mol, the content of PEG segment: 71-75wt%) to the L-lactide melt, stirring and heating to 120°C, continuously stirring at the speed of 100 r/min for 30min, and completely dissolving the poloxamer in the L-lactide melt to obtain the mixed solution;
S3. adding 10 parts by mass of erythromycin, continuously stirring at the speed of 115 r/min for 25min, and fully mixing;
S4. polymerization: adding 0.2 parts by mass of catalyst Sn(Oct)₂ to the material obtained from S3, heating to 150°C to promote the polymerization of L-lactide, letting the system stand for reaction for 1h, and then naturally cooling to ordinary temperature to obtain a mixture of erythromycin, polylactic acid microspheres, and the poloxamer;
S4. water washing: washing the mixture with water for 5 times, filtering and drying, and removing the poloxamer to obtain erythromycin-loaded polylactic acid microspheres.

### Example 15

S1. L-lactide melting: heating 100 parts by mass of solid L-lactide to 100°C until completely melted, and keeping the temperature for 10 min to obtain L-lactide melt;
S2. preparing the mixed solution: adding 40 parts by mass of the water-soluble polymer additive PEG-b-PPG-b-PEG (poloxamer 407, molecular weight: 9840-14600g/mol, the content of PEG segment: 71-75wt%) to the L-lactide melt, stirring and heating to 120°C, continuously stirring at the speed of 100 r/min for 30min, and completely dissolving the poloxamer in the L-lactide melt to obtain the mixed solution;
S3. adding 15 parts by mass of erythromycin, continuously stirring at the speed of 115 r/min for 25min, and fully mixing;
S4. polymerization: adding 0.2 parts by mass of catalyst Sn(Oct)₂ to the material obtained from S3, heating to 150°C to promote the polymerization of the L-lactide, letting the system stand for reaction for 1h, and then naturally cooling to ordinary temperature to obtain a mixture of erythromycin, polylactic acid microspheres, and the poloxamer;
S4. water washing: washing the mixture with water for 5 times, filtering and drying, and removing the poloxamer to obtain erythromycin-loaded polylactic acid microspheres.

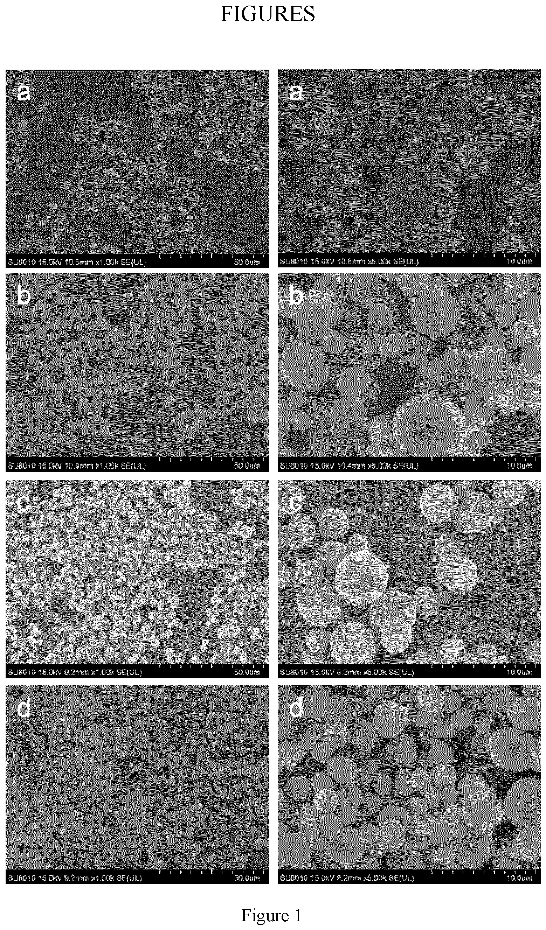

The polylactic acid microspheres prepared in Examples 2, 3, 4 and 5 are selected, and the morphology of the microspheres is observed with a scanning electron microscope to obtain SEM images as shown in Figure 1, wherein the left side is the SEM images with a magnification of 1000 times, and the right side is the SEM images with a magnification of 5000 times of the same sample. In Figure 1, a is the morphology of the polylactic acid microspheres prepared in Example 2; b is the morphology of the polylactic acid microspheres prepared in Example 3; c is the morphology of the polylactic acid microspheres prepared in Example 4; d is the morphology of the polylactic acid microspheres prepared in Example 5. It can be seen from Figure 1 that the polylactic acid microparticles prepared in Examples 2 to 5 are all spherical like particles.

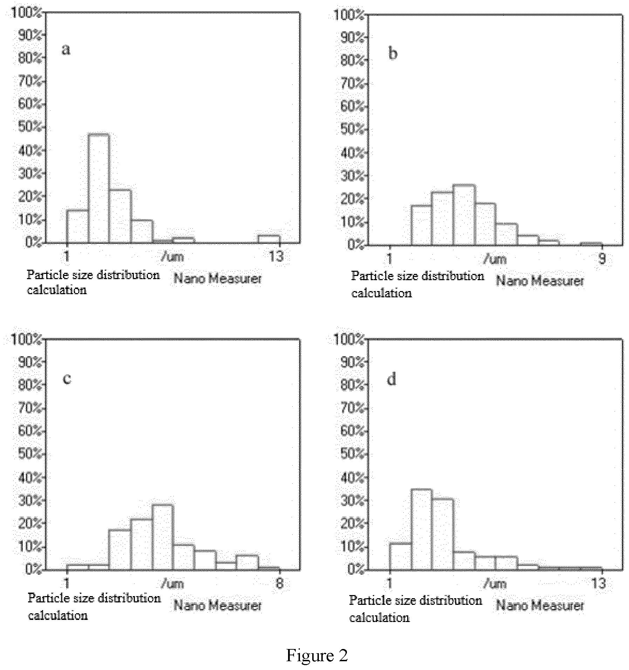

The particle size distribution of the polylactic acid microspheres prepared in Examples 2, 3, 4 and 5 is analyzed by a laser particle size analyzer, and the particle size distribution diagrams of the polylactic acid microspheres is obtained as shown in Figure 2. In Figure 2, a is the particle size distribution diagram of the polylactic acid microspheres prepared in Example 2, which shows the particle size is 1-13 µm, and the content of polylactic acid microspheres distributed within 1-4 µm is ≥ 88%. In Figure 2, b is the particle size distribution diagram of the polylactic acid microspheres prepared in Example 3, which shows the particle size is 2-9 µm, and the content of polylactic acid microspheres distributed within 2-6 µm is ≥ 85%. In Figure 2, c is the particle size distribution diagram of the polylactic acid microspheres prepared in Example 4, which shows the particle size is 1-8 µm, and the content of polylactic acid microspheres distributed within 3-6 µm is ≥ 80%. In Figure 2, d is the particle size distribution diagram of the polylactic acid microspheres prepared in Example 5, which shows the particle size is 1-13 µm, the content of polylactic acid microspheres distributed within 1-3 µm is ≥ 75%, and the content of polylactic acid microspheres distributed within 1-6 µm is ≥ 80%.

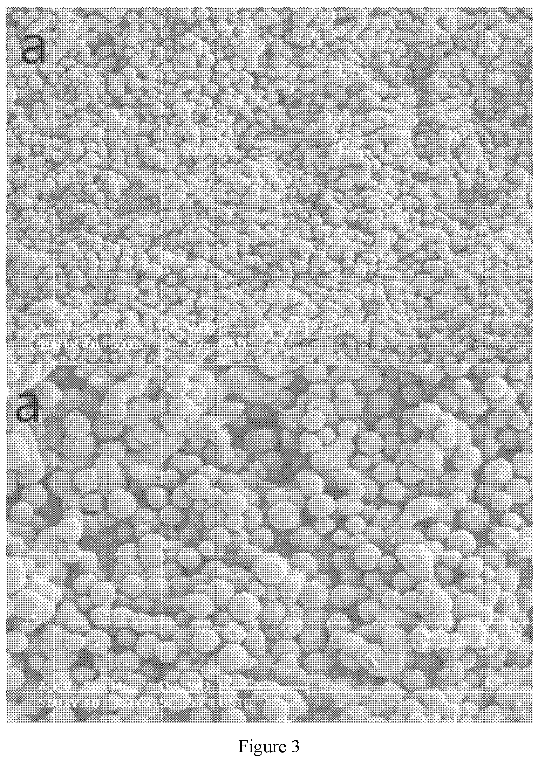

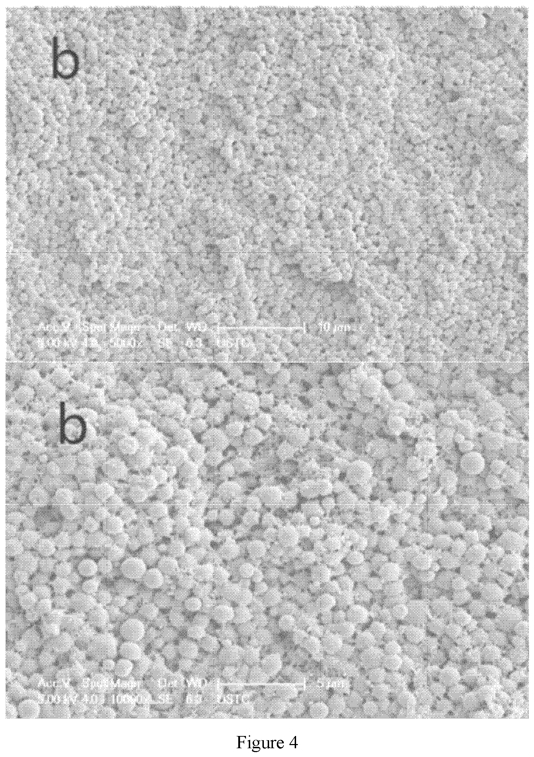

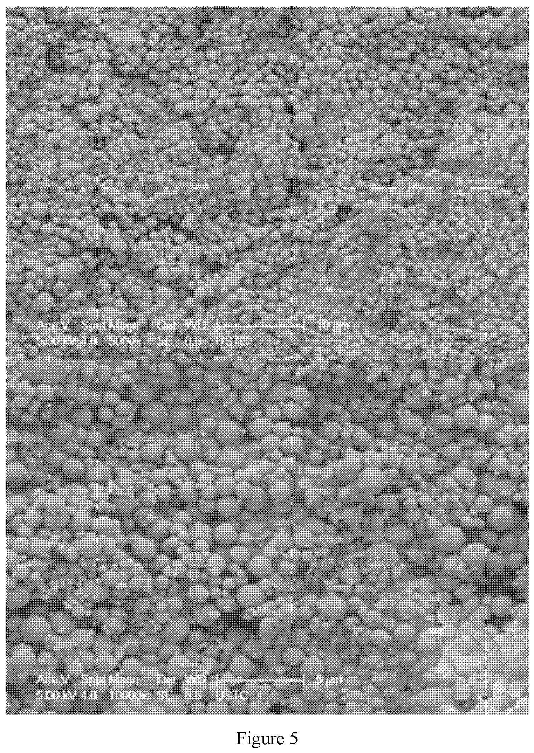

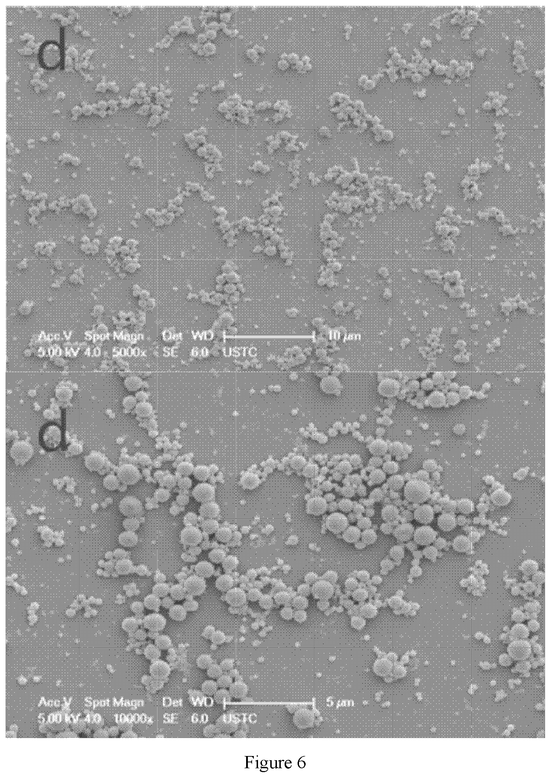

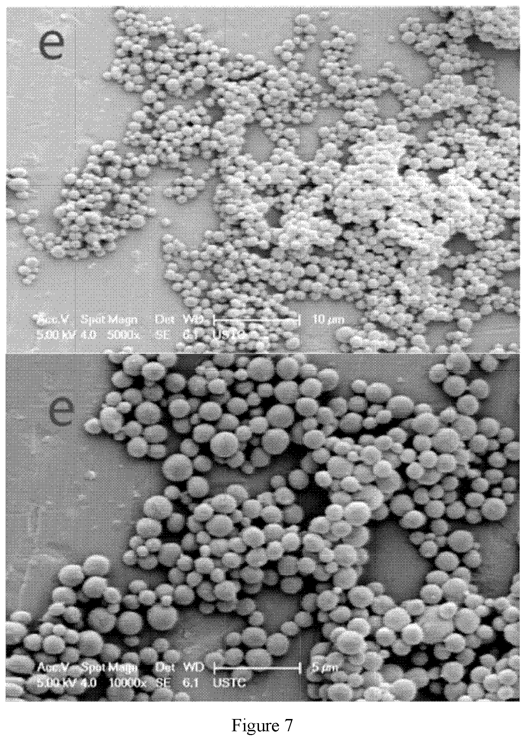

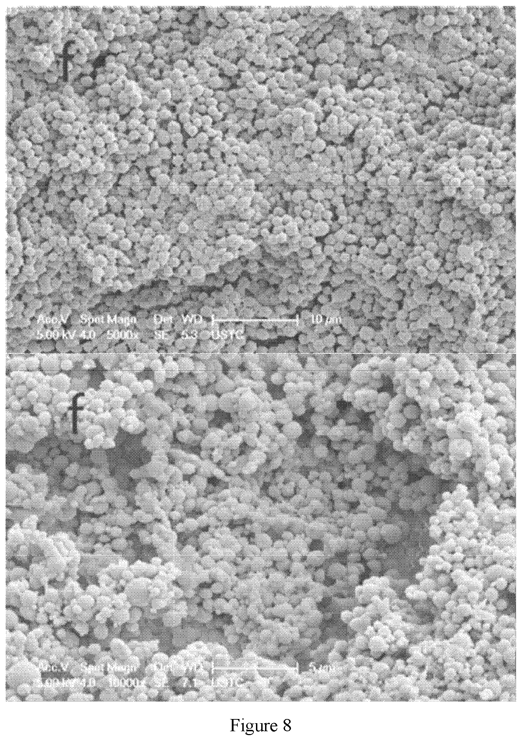

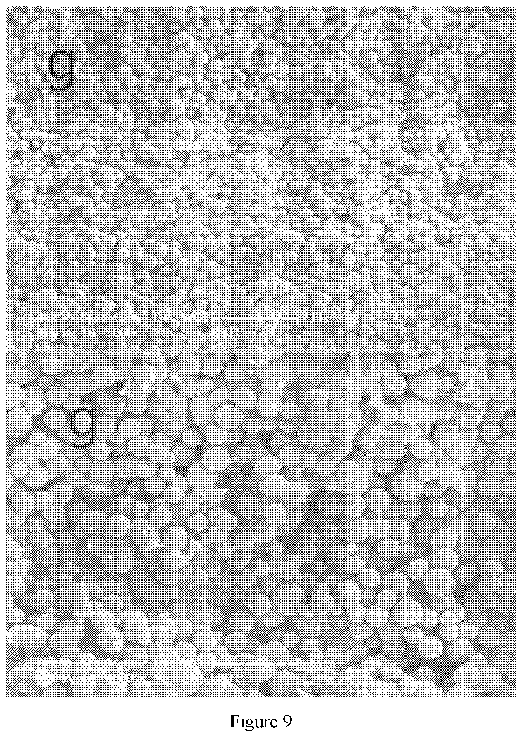

The morphology of the microspheres prepared in Examples 6-12 is observed with a scanning electron microscope, and the SEM images shown in Figures 3-9 are obtained. It can be seen that the microspheres prepared in Examples 6-12 are all spherical like particles.

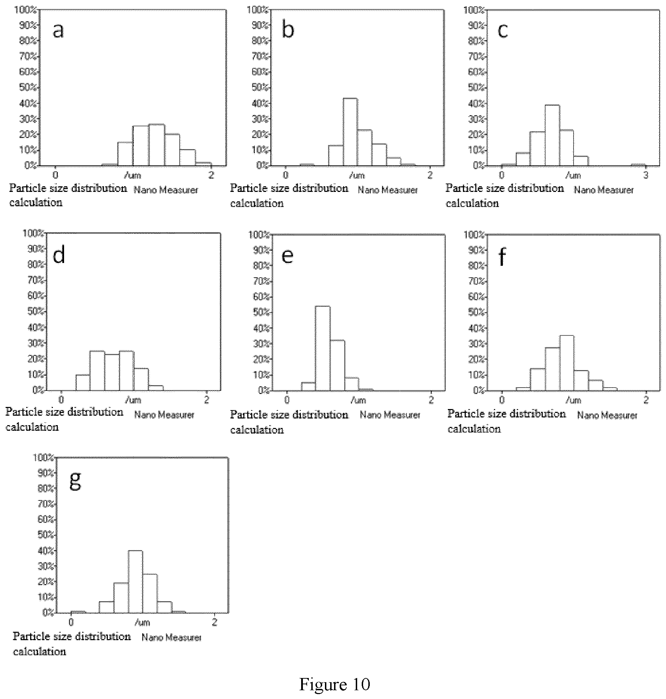

The particle size distribution of the microspheres prepared in Examples 6-12 is analyzed by the laser particle size analyzer, and the particle size distribution diagrams of the microspheres are obtained as shown in Figure 10. It can be seen that the particle size of the microspheres prepared in Examples 6-12 is 1-3 µm, and the content of the microspheres with the particle size distribution of 1-2 µm is ≥ 80%.

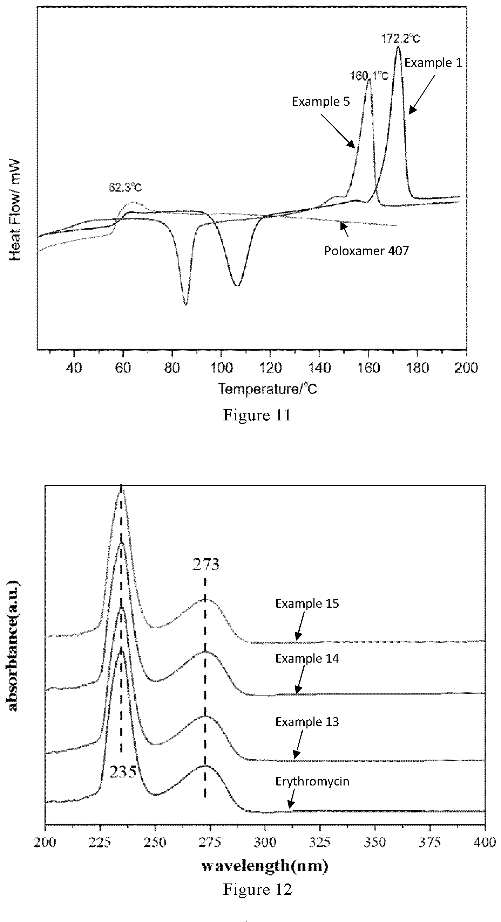

The polylactic acid microspheres prepared in Example 1 and Example 5 and the pure poloxamer sample are selected for thermal analysis to obtain DSC curves as shown in Figure 11. The absorption peak at 172.2°C of the sample of Example 1 represents the melting temperature of polylactic acid. The temperature corresponding to the melting absorption peak of the sample of Example 5 is 160.1°C, which is significantly lower than that of the sample of Example 1. This may be due to the fact that the poloxamer participates in the polymerization process and forms a graft with the polylactic acid, which destroys the crystal integrity of the polylactic acid and lowers the melting point.

Figure 12 shows the UV absorption spectra of the samples of Examples 13-15, and the characteristic peak at 235 nm corresponds to the absorption peak of erythromycin. According to the ratio of the absorption peak intensity of pure erythromycin to that of the microspheres at 235 nm, the encapsulation efficiencies of erythromycin in the microspheres prepared in Examples 13, 14 and 15 are calculated to be 42%, 45% and 48%, respectively.

The above embodiments are only illustrative of the principle and efficacy of the present invention, and are not intended to limit the present invention. Any person skilled in the art can modify the microspheres of the above embodiments to obtain biodegradable polymer microspheres with certain functions without departing from the spirit and scope of the present invention, which should still be covered by the claims of the present invention.

## Claims

1. A biodegradable polymer microsphere, wherein the biodegradable polymer microsphere comprises a biodegradable polymer, and a particle size of the biodegradable polymer microsphere is ≤ 20µm.

2. The biodegradable polymer microsphere of claim 1, wherein the biodegradable polymer is an aliphatic polyester, such as polylactic acid, polycaprolactone, polyglycolide acid, lactide-caprolactone copolymer, lactide-glycolide copolymer, caprolactone-glycolide copolymer, or lactide-caprolactone-glycolide copolymer.

3. The biodegradable polymer microspheres of claim 1, wherein the particle size of the biodegradable polymer microsphere ranges from 0.5µm to 15µm; preferably, a number of the biodegradable polymer microsphere with a particle size between 1µm to 6µm accounts for more than or equal to 80% of a total number of the biodegradable polymer microsphere.

4. The biodegradable polymer microsphere of claim 1, wherein the biodegradable polymer microsphere further comprises an antioxidant; preferably, the antioxidant is selected from one or more of antioxidant 168, antioxidant 1010, antioxidant 1098, antioxidant 626, and antioxidant THP-24; preferably, in the biodegradable polymer microsphere, the mass ratio of the biodegradable polymer to the antioxidant is 100: (0.1-1).

5. The biodegradable polymer microsphere of claim 1, wherein the biodegradable polymer microsphere further comprises a functional material, the functional material is preferably selected from an inorganic functional powder and a drug, the inorganic functional powder is preferably selected from one or more of graphene, graphite, carbon nanotubes, and magnetic nanoparticles, and the drug is preferably selected from one or more of erythromycin, ibuprofen, clindamycin, amoxicillin, cefradine, acetylspiramycin, azithromycin and oryzanol; preferably, in the biodegradable polymer microsphere, the mass ratio of the biodegradable polymer to the functional material is 100: (1-20).

6. A method for preparing the biodegradable polymer microsphere of any one of claims 1-5, wherein the method comprises a step of polymerizing a biodegradable polymeric monomer in a mixed solution comprising the biodegradable polymeric monomer, a water-soluble polymer, and a catalyst.

7. The method of claim 6, wherein the biodegradable polymeric monomer is selected from a monomer of an aliphatic polyester, preferably selected from one or more of aliphatic lactides and aliphatic lactones, such as one or more of lactide, caprolactone, and glycolide.

8. The method of claim 6, wherein the water-soluble polymer is selected from one or more of polyoxyethylene-polyoxypropylene ether block copolymer, polyacrylamide, polyvinylpyrrolidone, polyethylene glycol, polypropylene glycol, poly(vinyl alcohol), poly(acrylic acid), poly(methacrylic acid) and methyl cellulose; and/or in the mixed solution, the mass ratio of the biodegradable polymeric monomer to the water-soluble polymer is 100: (20-300).

9. The method of claim 6, wherein
the catalyst is selected from a metal, a Lewis acid, a Lewis base, an organometallic compound and a metal salt, such as one or more of Fe, Cu, Zn, Co, an alkali metal salt, a Schiff base, dibutyl magnesium, an aluminum alkoxide, Ti[OCH(CH₃)₂]₄, Sn(Oct)₂, SnCl₄, Sn(C₆H₅)₄, Sn(Oct)₂/CH₃(CH₂)₁₁OH and Sn(Oct)₂/P(C₆H₅)₃; and/or
in the mixed solution, the mass ratio of the biodegradable polymeric monomer to the catalyst is 100: (0.1-2).

10. The method of claim 6, wherein the mixed solution further comprises an antioxidant and/or a functional material.

11. The method of claim 6, wherein the reaction temperature of polymerization is 90-180°C.

12. The method of claim 6, wherein the method comprises the following steps:
S1. preparing a liquid monomer: taking a biodegradable polymeric monomer that is in liquid form at ordinary temperature for later use; or heating and melting a biodegradable polymeric monomer that is in solid form at ordinary temperature to obtain a liquid monomer;
S2. preparing the mixed solution: adding the water-soluble polymer into the liquid monomer, and completely dissolving the water-soluble polymer into the liquid monomer to obtain the mixed solution;
S3. polymerization: adding the catalyst into the mixed solution, letting the solution stand at reaction temperature to promote polymerization of the monomer, and obtaining a mixture of the biodegradable polymer microsphere and the water-soluble polymer;
S4. post-treatment: post-treating the mixture to obtain the biodegradable polymer microsphere.
